(19)

**Europäisches Patentamt**

**European Patent Office**

**Office européen des brevets**

(11)   **EP 3 892 199 A1**

(12)   **EUROPEAN PATENT APPLICATION**

(43) Date of publication:
**13.10.2021  Bulletin 2021/41**

(51) Int Cl.:
***A61B 6/00*** *(2006.01)*

(21) Application number: **20168199.6**

(22) Date of filing: **06.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AGFA NV**
**2640 Mortsel (BE)**

(72) Inventors:
• **THOMA, Ralph**
**81539 München (DE)**
• **NEBOSIS, Rainer**
**81539 München (DE)**

(74) Representative: **Pieters, Dirk Paul Oscar**
**Agfa NV**
**Intellectual Property Department**
**Septestraat 27**
**2640 Mortsel (BE)**

(54) **METHOD AND DEVICE FOR ADJUSTING THE GEOMETRIC AND DOSE PARAMETERS FOR AN X-RAY ACQUISITION**

(57)     The present invention provides a method and a device that is used to optimize both the exposure settings and the geometric configuration of an X-ray imaging device based on a pre-shot image. The device is an X-ray absorbing mask that comprises a particular set of features that allows for the acquisition of a calibration pre-shot image that comprises information that allows to optimize both the geometry as the exposure settings for a subsequent main-shot acquisition.

Fig. 3

## Description

Technical Field

The present invention relates generally to a method and a device to optimize all parameters concerning the configuration of an X-ray acquisition. The method involves the application of a specially designed radiation blocking mask that is inserted between the X-ray source and the patient during the acquisition of a low-dose calibration pre-shot image. A subsequent analysis of the image allows the calculation of eventual deviations from an ideal alignment of the detector with the source, as well as the calculation of the optimal X-ray parameter settings for the X-ray generator.

Background of the invention

It has been a long standing problem in conventional radiography to accurately determine in advance the various parameters that are involved in the configuration of a radiography system for a certain exposure and for a particular patient. Especially in radiography configurations where the respective positions of the patient, detector and source are not accurately known, this uncertainty may lead to need to make certain assumptions about the configuration and for instance the size of the patient by the operator.

For instance, it is still a problem to measure the position and orientation of an X-ray panel with respect to an X-ray source for so-called free X-ray exposures, which are exposures where the detector is not positioned in a table or wall stand bucky (with known coordinates) as it is not possible to position the patient on such table or against such a wall stand. An example of such a case is where a patient is lying in a bed such that an imaging sensor has to be positioned by an operator using his skills to make sure that both the detector and the region of interest end up in the X-ray beam during the acquisition.

Another aspect of any radiography exposure is that it is hard to make good estimates of the X-ray exposure settings under all circumstances. The operator may not have always a good view on the patient thickness of the body part imaged by the X-ray detector. This thickness is however an essential parameter to be considered when determining the optimal X-ray exposure settings.

In conventional radiography, the beam of an X-ray source (in most cases an X-ray tube) is modified or shaped to optimally expose the patient tissue subject to examination to render an optimal image quality result, while minimizing the patient exposure to radiation. For this reason, it is common to limit the size of the beam as much as possible to the region of interest to be examined in order to limit the patient dose, but also to limit image quality deterioration due to scatter radiation. The part of the X-ray beam exposing the region of interest of a patient is partially attenuated by the tissue encountered by the beam on its path to the imaging sensor, and forms a latent image that is accumulated (CR-image) or integrated (DR-image) in a detector sensitive to X-rays.

The exposure settings fully determine the quality of the X-ray beam and have an important impact on the resulting image. The exposure settings are essentially determined by the type of exam to be carried out, and depend on the following: type of examination (soft tissue examination require different settings than the examination of bony structures), age of the patient (pediatric or not), specific acquisition geometry SDD (source detector distance), ODD (object detector distance) and incidence angle of the beam) and thickness of the patient. The exposure settings are defined in terms of mA (electric current passed through the anode of the X-ray tube), s (exposure duration in seconds), and kV (tube voltage in kilo-volts).

In fixed radiography installations, the acquisition geometry is relatively easy to be determined since the degrees of freedom of the movements of the different modality components are defined by the modality design and the movements of the individual components can be easily tracked by measuring their displacements. The exact locations in space of the main determining components for the acquisition geometry (namely the X-ray source, the image detector and table surface) can be relatively easily calculated by tracking or measuring the movements of those components relative to reference positions. Digital readings of the displacements or rotations of a C-arm gantry can unambiguously define the emission point and inclination angle of the X-ray beam, for instance. In a typical fixed radiography installation, also the location of the image detector can be unambiguously located because of the fact that it resides in a so-called detector "bucky" of which the location in the table is predetermined or can be easily measured.

Certain methods in the art describe methods to resolve the above mentioned problems partly, but not completely. Although that it would be highly preferable to resolve the above mentioned problems without the need for a supplementary pre-shot acquisition that causes a small amount of additional dose to the patient, the most comprehensive solutions ensuring the most accurate results do imply the use of a calibration pre-shot image (or scout image) obtained from such a pre-shot acquisition. It speaks for itself that dose considerations come into play before such methods may be accepted as a viable solution.

Studies indicate that nearly 50% of all X-ray acquisitions are either overexposed (27%) or underexposed (23%) - in which case 17% of these underexposed images caused a retake of the image. It has been found from bed exam studies in ICU's that when an overexposure happens, the dose applied is more than 50% higher than the optimum dose for the intended exposure. These unnecessary doses (from re-takes and overexposure) can be reduced or even eliminated when using a pre-shot image to calculate the optimal generator settings. This however comes at the cost of the dose involved in such a pre-shot.

The patient dose involved for the acquisition of a suitable pre-shot image typically amounts to 5% of the dose of a diagnostic image. This limited dose ensures a sufficient image quality in the exposed areas of a pre-shot image. Moreover, in solutions described in the art, an X-ray absorbing mask is often applied which further reduces the dose reaching the patient. The overall dose of a pre-shot image may thus even become lower than 1% of the typical dose of a diagnostic image, which then becomes a solid argument in favour of systematic use of a pre-shot to optimize the various acquisition settings. This small additional dose largely compensates the total amount of unwanted dose incurred due to re-takes and applying incorrect dosages.

US 9615803 B2 describes a method to determine the optimal exposure settings for an X-ray imaging device based on the acquisition and analysis of a pre-shot image (also called scout image). Such a pre-shot image is a low-dose acquisition involving only a fraction of the dose used for a conventional radiography, and that is taken under the same circumstances as the actual main-shot acquisition. The pre-shot dose allows to obtain a measurement on the actual X-ray imaging device providing sufficient data to calculate the necessary exposure settings for a subsequent "main-shot" acquisition (at full dose). The disclosure does not report on the use or possible use of an absorbing mask.

US 7488107 B2 discloses a method and apparatus to detect and correct alignment errors in an X-ray system, based on the acquisition of a pre-shot image and the application of a specifically designed absorbing mask. The projection of the mask provides information regarding a position of the detector with respect to the source. Based on the captured projection of the mask, it is possible to calculate the inclination, rotation and distance of the imaging sensor from the source.

There is however no disclosure of a system or method that is capable of obtaining both the optimal exposure settings and the geometric alignment corrections based on a single acquired pre-shot image. Solving this problem is the object of this invention.

Another object of the invention is to minimize the deposited dose during the acquisition of the pre-shot image, but such that the above measurements, analysis and correction still can be performed based on a single pre-shot image.

Summary of invention

The present invention provides an X-ray absorbing mask for acquiring a calibration pre-shot image by an X-ray imaging sensor of an X-ray imaging device when exposed by an X-ray source, the X-ray absorbing mask being applied between the X-ray source and a patient, the X-ray absorbing mask comprising a sheet of homogeneous thickness and a plurality of recesses organized in zones, wherein at least three spatially separated zones that each comprise a set of recesses that are arranged in at least two spatial directions and which produce an intensity modulation on said X-ray imaging sensor and that are suitable to measure the local magnification, at least one zone comprising a recess having a width and height of at least 1% of the width and height of said X-ray absorbing mask that generates an X-ray shadow of the patient in said calibration pre-shot image, and the surface area of recesses making up at most 30% of the surface area of the X-ray absorbing mask.

The present invention intends to overcome the problems and limitations of the solutions described in the prior art.

In the context of this invention, an X-ray absorbing mask has to be understood as a radio-opaque plate with recesses or holes that allow X-ray photons to pass through in a defined pattern. Radio-opacity is the relative inability of the X-ray portion of electromagnetic spectrum to pass through a particular material. Such an X-ray absorbing mask intends to cast an X-ray shadow of the pattern cut out of the plate onto an object, in our case a patient behind which an X-ray imaging sensor is positioned, such that the X-ray shadow may be analyzed. The X-rays that are passing through the recesses of the mask, and that are not blocked by the plate material, may be partially absorbed by the tissue of the patient before hitting the X-ray imaging sensor.

The X-ray absorbing mask may be applied in an X-ray imaging device that comprises at least an X-ray source and an X-ray imaging sensor. It is intended to shape the X-ray beam such that a pattern is projected onto an X-ray imaging sensor. This pattern can then be analysed in the resulting digital image. The mask should be installed at a known position in the configuration with respect to the X-ray source of the X-ray imaging device. An obvious position for positioning the mask is close to the exit of the X-ray source, and thus in practice attached to the X-ray beam limiting device (or collimator) that is mounted to the X-ray tube itself. The collimator already shapes the beam such that a trimmed and controlled X-ray beam field exits the X-ray tube.

When applying the X-ray absorbing mask in the collimated X-ray field during an image acquisition, the recorded image by the X-ray imaging sensor will comprise two types of information; on one hand there are the image quality data that are intrinsically present in those portions of the image that passed through the recesses of the absorbing mask and that may be used to analyze the image quality of the acquired image under the pre-shot conditions. The analysis of these data can be used to optimize and adjust the X-ray exposure settings (kV, mAs) of the beam for the main-shot.

At the other hand, there is geometric alignment information present in the pre-shot image from the projection of the absorbing mask on the X-ray imaging sensor. The specific shapes of the recess pattern provided in the X-ray absorbing mask allow to calculate the location and orientation of the X-ray imaging sensor with respect to the location of the absorbing mask and X-ray source.

The X-ray absorbing mask has to be designed such that the above two types of information can be obtained from a single pre-shot. Therefore, a number of zones with specific shapes of recesses are foreseen on the surface of the sheet of material.

In the context of this invention, a zone has to be understood as a contiguous portion of the X-ray absorbing mask surface. Each zone comprises a number of recesses that are grouped such that particular aspects of the X-ray beam quality and/or geometry can be measured at different locations of the acquired image.

The invention relies on the acquisition of a calibration pre-shot image (or pre-shot image), which is an image that is acquired by applying a low dosage X-ray beam to the patient using the X-ray absorbing mask as described above. The pre-shot image is (obviously) acquired before the acquisition of the main-shot image. The main-shot image is the ultimate diagnostic image of which the acquisition settings may be optimized and adjusted as a consequence of the results of the analysis of the pre-shot.

Since the pre-shot is not used for diagnosis, it may be considered as a supplementary dose that is attributed to the patient. For this reason, the actual pre-dose delivered to the patient should be limited as much as possible. While the pre-shot dose already corresponds to only a fraction of the main dose, the absorbed dose by the patient may be further reduced by limiting the amount of recessed surface of the X-ray absorbing mask. The amount of obstructed X-ray beam with respect to the unobstructed dose area should ideally be maximized. This may be achieved to design the absorbing mask such that the recessed surface on the absorbing mask is minimized. Selected areas which do not represent the most desirable locations for the above mentioned zones therefore should comprise closed area.

The invention is advantageous in that it allows to both optimize the dose parameters as well as the geometric parameters by using a single pre-shot. The resulting pre-shot image allows for the analysis and calculation of the optimal X-ray settings, as well as the calculation of the geometric corrections that would be required to adjust the geometric configuration of the imaging modality to obtain the optimal acquisition geometry for the main-shot exposure (i.e. perfect orthogonal alignment of the X-ray imaging sensor perpendicular to the axis of the X-ray beam).

At the same time, is the invention advantageous in that it limits the absorbed dose by the patient as much as possible, by means of limiting the open or recessed area of the X-ray absorbing mask by design.

Specific examples and preferred embodiments are set out in the dependent claims.

Further advantages and embodiments of the present invention will become apparent from the following description and drawings.

Brief description of drawings

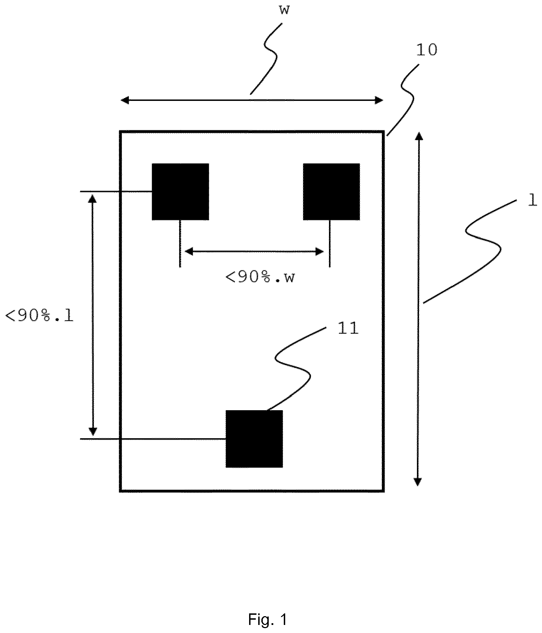

Fig. 1 provides a design of a preferred embodiment of an X-ray absorbing mask of the invention, wherein three identical zones [11] are spatially distributed on the masks surface. [*w*] indicates the width and [*l*] indicates the length of the absorbing mask. The optimal horizontal, respectively vertical, distance between the centres of two zones is 90% of the entire mask width, respectively 90% of the entire mask length as indicated in the drawing.

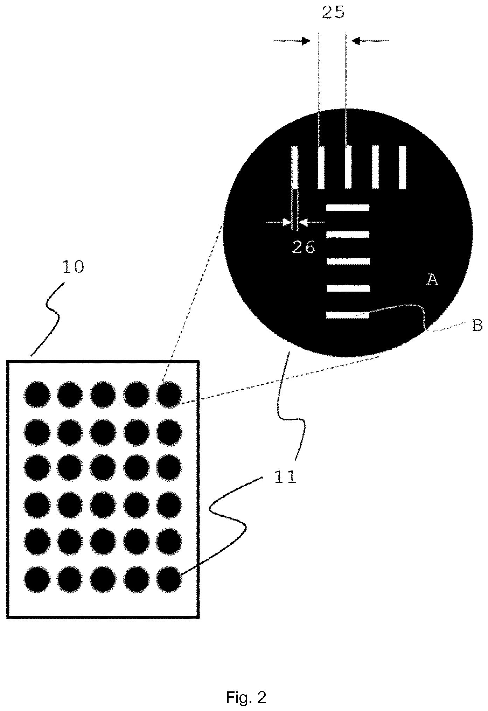

Fig. 2 shows another layout for a design of an X-ray absorbing mask [10] of the invention, wherein a series of spatially distributed circular zones [11] are depicted. The drawing also shows a magnified layout of one circular zone wherein two series of parallel recesses [B] are orthogonally arranged in X-ray absorbing material [A]. The distance between two consecutive recesses [25] is shown as well as the width of a single recess [26].

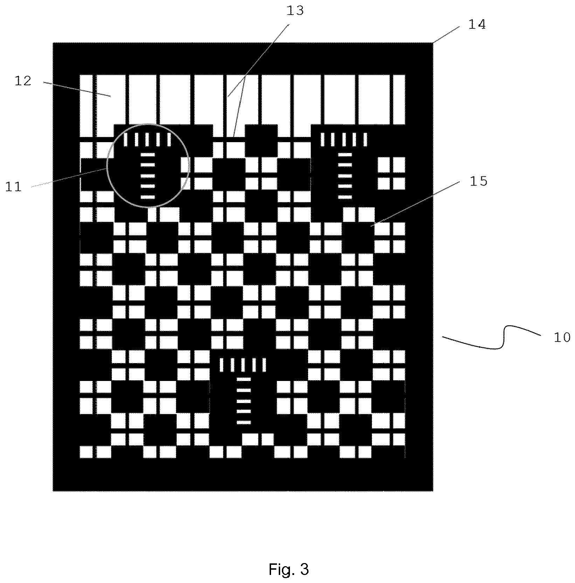

Fig. 3 shows a preferred embodiment of the X-ray absorbing mask [10] of the invention. In this particular design, the identical zones [11] are integrated between other functional structures. Only one of the zones [11] is marked, however three identical structures are present. Large open zones or structures [12] are provided to allow a special area of interest to be imaged by the pre-shot for careful evaluation. Other structural features are marked in the drawing; [14] is the support frame or rim that ensures structural stability of the entire absorbing mask, [15] is an area that consists of the X-ray blocking material (sheet material) and that serves as a dose reduction structure. Further, there are multiple small support structures [13] present that keep the functional structures (the support frame [14], the zones [11] and the dose reduction structures [15]) together.

Description of embodiments

In the following detailed description, reference is made in sufficient detail to the above referenced drawings, allowing those skilled in the art to practice the embodiments explained below.

The embodiment of the X-ray absorbing mask of the invention as depicted in Fig. 1 is intended to be positioned between the X-ray source and the patient, and this at a distance of at most 50% of the distance between mask and X-ray source. In practice, the X-ray absorbing mask will be positioned in a dedicated position close to the X-ray source.

Fig. 1 represents a drawing of a preferred embodiment of an X-ray absorbing mask of the invention, wherein the optimal distribution of three identical zones is indicated; wherein [*l*] and [*w*] are respectively the length and width of the absorbing mask. The shape and features of the mask will be magnified and projected onto the X-ray detector. An ideal arrangement of the zones across the mask surface can be achieved when the minimal horizontal distance between the centres of two zones is 90% of the width [*w*] of the mask or less. The same applies in the vertical direction. Besides this, a sufficiently large support frame or rim should be foreseen around the zones in order to ensure mechanical stability of the X-ray absorbing mask. Additional zones can be arranged in-between the three zones [11] in figure 1.

A large distance between two neighbouring zones allows to easily compare the sizes and shapes of the projections of the zones onto the X-ray imaging sensor, such that the orientation of the detector can be calculated. In case that the zones are spatially separated as much as possible on the mask, the projected images of the zones will show the largest differences and thus will the calculations that are based on the size and shape measurements be the more accurate. On the other hand, all zones must lie within the collimation area which may be smaller than the entire detector area. Therefore, additional zones in-between the 3 indicated zones may be advantageous.

The zones ideally have identical shapes of patterns of recesses in order to allow an easy comparison between the measured projected patterns at the different mask locations. The features in the zones that are implemented as recesses may be linear, or may have different shapes, such as squares rectangles, pinholes, circles or alike. The recesses may be a series of parallel linear recesses, or may be for instance squared and arranged in a QR-coded pattern. It is however important that the series of recesses in each zone produce a detectable intensity modulation on the X-ray imaging sensor. This requirement is met when the smallest pattern on the mask of which the image is casted onto the X-ray imaging sensor is smaller than the size of a sensor pixel when projected on the X-ray imaging sensor. The pattern should be arranged in at least two directions, allowing its distance to the X-ray source to be calculated based on the measured local magnification M of this pattern in the image produced by the X-ray imaging sensor. The observed local magnification M of the pattern on the detector has a relation to distance of the X-ray imaging detector to the source and the mask.

$$M = \frac{SID}{source-to-mask\ distance}$$

Wherein the SID, is the source to X-ray image sensor distance.

The X-ray absorbing mask is constructed out of a solid and rigid sheet material that ideally absorbs at least 90% of the incident radiation. The recesses represent areas where the mask material is fully or partially removed. It is important that the amount of absorbed radiation significantly differs between the recessed areas and the solid or non-recessed areas area. This difference in absorption is preferably at least 50%.

Alternative embodiments may be envisaged that for instance comprise more than the minimum of three zones.

Fig. 2 gives an example of such an embodiment of an X-ray absorbing mask [10] wherein thirty zones [11] are arranged at more or less equal distances from each other. In the Fig. 2 an enlarged detail of such a zone [11] is depicted.

The zone of the embodiment comprises a set of periodic structures, namely a series of five horizontally arranged and five vertically arranged longitudinal slits or recesses [B] that are carved out of a solid sheet material [A]. The width [26] of the recesses and the respective interspacing distances [25] generate an intensity modulated pattern in a calibration pre-shot image in two orthogonal directions. The casted X-ray pattern that is captured in the pre-shot image and can then subsequently analysed.

For example, the projected pattern pitch [25] can be measured in the pre-shot image, which provides information about magnification of the projected patterns. This magnification factor then can be used to calculate the actual SID (source detector distance) at the time of the pre-shot, provided that the distance between the X-ray source and the X-ray absorbing mask is known.

In case that the magnification factors of the different zones show differences, this means that the different zones have different SID's at the level of the imaging sensor. In other words, the different projections of the zones do not have the same distances from the X-ray source, and thus the X-ray detector was not oriented fully orthogonally to the X-ray beam axis at the time of the pre-shot. Based on the measurement of the differing magnification factors for the zones, the different SID's for each zone -and thus the orientation of the X-ray detector surface- can thus be calculated. The calculated orientation, can subsequently be used to correct for the alignment deviation of the X-ray imaging sensor with the X-ray beam axis.

Ideally, the zones have similar or identical shapes that are duplicated multiple times on the sheet surface. The shapes and patterns should preferably be easily detectable by image processing algorithms. As an example it is advantageous to shape these zones as a QR code which then could serve multiple purposes; a QR allows easy detection of its presence, it allows the easy localisation of its centre, and it allows to encode additionally any desired digital information (that could for instance identify the zone and its assumed location on the absorbing mask). QR codes are easily located and detected by state of the art detection algorithms.

Alternatives for types of patterns or shapes that may be used in the zones are for instance, bar codes, a set of orthogonally arranged series of parallel lines, or a non-periodic pinhole structure with known locations of holes.

The preferred embodiment that is represented in Fig. 3 combines the necessary functional features into one single X-ray absorbing mask to allow to make the combination of adjustments based on a single pre-shot acquisition. The embodiment comprises the necessary structures to 1) measure and adjust geometric deviations from the correct configuration (by means of the at least three identical zones [11]), 2) measure the image quality based on parts of the image data that reach the detector through the large open zones [12], and 3) reduce the total delivered dose to the patient delivered by the pre-shot.

Additional structures such as the mask frame [14] and a number of small support structures [13] provide the required rigidity for the entire mask.

Considering that the recessed area of the embodiments of the invention should cover only about 10% of the masks' complete surface, the total delivered dose to the patient during the pre-shot (when the X-ray absorbing mask is in place) is at around 20% of the total dose of the pre-shot (considering that 90% of the surface still allows 10% transmission of the X-rays). Therefore, can the design of the mask further reduce the delivered dose to the patient while enjoying the advantages of the analysis of a single pre-shot acquisition.

The following procedure can be performed to work the invention. In a preparative step, the X-ray absorbing mask is inserted between the X-ray source and the X-ray imaging sensor to acquire the pre-shot image or scout image. The pre-shot exposure settings are limited to produce around 5% of the dose of a diagnostic image in order to limit the dose for the patient. The digital pre-shot image is read out from the imaging modality for further analysis. The mask is then removed to acquire the diagnostic image after performing the geometric and dose setting adjustments that are obtained and calculated in the following steps.

The digital pre-shot image is first analyzed to calculate any geometric adjustments as follows:

1- the location in the image of the periodic structures that are arranged in zones is determined by for instance pattern recognition, via Fourier transformation or by triangulation of the pinhole structure (in the case that pinholes are present),

2- next, the spatial frequency of the periodic structures in the located zones is measured. Alternatively, the size of the QR code like structure may be estimated or the distances between the pinholes are calculated by means of triangulation.

3- the measured spatial frequency of the periodic structures is converted into a distance between the X-ray source and each individual periodic structure. Since the spatial frequency is a function of the pattern pitch [25] of the periodic structures in the mask and the magnification M; the magnification M can be calculated out of the measured spatial frequency in the pre-shot image and the known spatial frequency of the pattern pitches in the mask. Since the magnification M describes the relation of the distances between the X-ray source to respectively the mask and the imaging sensor, the distance between the X-ray source and the imaging sensor can be calculated. Based on the same principle, the SID (source to detector distance) can be calculated out of the QR pattern size or the distances between pinholes.

4- the SID applied during the pre-shot is calculated from the (weighted) mean of the calculated distances between the X-ray source and the periodic structures,

5- the orientation of the imaging sensor (tilt) can be calculated from the different source to detector distances for the different zones detected at the level of the detector. In case that different distances are calculated between the source and the multiple zones, this means that the X-ray imaging sensor is not fully oriented orthogonally with respect to the X-ray beam axis. A rotation of the imaging sensor around the X-ray beam axis can be detected by measuring the rotation angle of the projected pattern with respect to the imaging sensor edges.

6- calculate the geometric adjustments that may be applied to the X-ray source or the imaging sensor in order to achieve an optimal (orthogonal) orientation of the X-ray source axis with respect to the imaging sensor.

7- calculate the adjustments to the SID (source to imaging sensor).

8- evaluate the position of the region of interest of the patient within the X-ray beam by evaluating the visible content of the pre-shot image.

9- instruct the user on how to properly adjust all positions and orientations of the X-ray source, the collimator settings, the patient and the imaging sensor. Alternatively apply these settings automatically by means of motorized movement of the X-ray source and/or imaging sensor.

Secondly, the same digital pre-shot image is analyzed to evaluate the X-ray quality settings of the pre-shot in order to adjust them for the subsequent main-shot as follows:

1- identify the regions in the pre-shot image wherein the patient is exposed (i.e. where the X-rays are not shielded by the absorbing sheet material of the mask): the unshielded area.

2- calculate statistical image quality values based on the pixel values in the unshielded area:

a- mean pixel value
b- minimum and maximum values by using small sub-regions (i.e. by using binned pixels; where the binning sizes are chosen between 2x2 and 10x10 pixels)
c- signal-to-noise ratio
d- contrast-to-noise ratio

e- scatter-to-primary ratio

3- calculate the optimum exposure parameters for the main-shot by means of one of the following methods:

a- Predefine or select a desired mean pixel value for the main-shot by applying an absorption model as a function of the tube voltage and mAs setting that is described in the art.

b- Predefine or select a desired signal-to-noise ratio or contrast-to-noise ratio for the main-shot by applying an absorption model as a function of the exposure settings (kV, mAs) that is described in the art.

c- Further optimize the method described under a- and b- by excluding parts of the image data from the calculation based on the mean pixel value (so to exclude areas where for instance bone tissue is present)

4- instruct the user to adjust the exposure settings of the modality to the calculated exposure settings, or alternatively transfer the calculated exposure settings to the imaging modality system for automatic programming of the main-shot.

Because of the fact that the main-shot image (diagnostic image) is acquired at a later point in time compared to the pre-shot image (scout image), it is important that the modality configuration (including the position of the patient) is not involuntarily altered. Between the pre-shot and the main-shot, the configuration at least has to be altered in the sense that the absorbing mask has to be removed. Optionally, and subject to the aforementioned calculations, other modality parameters and component positions may be voluntarily altered.

In order to avoid involuntary changes to the main-shot configuration, an acceleration sensor in the panel can be used to detect imaging sensor motion after the pre-shot image was taken. In the case that a motion of the imaging sensor is observed, the additional displacement has to be taken into consideration when calculating the final modality configuration, or at least need the operator be alerted about the involuntary motion of the component. Additionally, can a visible light camera be used to detect patient motion after the pre-shot image was taken, and to respond according to the same principles as explained above.

**Claims**

1. X-ray absorbing mask [10] for acquiring a calibration pre-shot image by an X-ray imaging sensor of an X-ray imaging device when exposed by an X-ray source, the X-ray absorbing mask being applied between the X-ray source and a patient, the X-ray absorbing mask comprising a sheet of homogeneous thickness and a plurality of recesses organized in zones [11], wherein:

- at least three spatially separated zones [11] that each comprise a set of recesses [B] that are arranged in at least two spatial directions and which produce an intensity modulation on said X-ray imaging sensor and that are suitable to measure the local magnification,
- at least one zone comprising a recess having a width and height of at least 1% of the width and height of said X-ray absorbing mask that generates an X-ray shadow of the patient in said calibration pre-shot image, and
- the surface area of recesses making up at most 30% of the surface area of the X-ray absorbing mask.

2. X-ray absorbing mask of Claim 1, wherein the at least three spatially separated zones comprise a set of recesses arranged as a set of two orthogonally arranged bar-codes.

3. X-ray absorbing mask of Claim 1, wherein the at least three spatially separated zones comprise a set of recesses arranged as a QR code.

4. X-ray absorbing mask of Claim 1, wherein the at least three spatially separated zones comprise a set of recesses arranged as a pinhole pattern.

5. X-ray absorbing mask of any of the previous claims, wherein said sheet consists of a material or combination of materials that attenuates an X-ray beam at the applied thickness by at least 90%.

6. X-ray absorbing mask of any of the previous claims, comprising a support frame [14].

7. X-ray absorbing mask of any of the previous claims, wherein said X-ray absorbing mask is positioned at a distance from the X-ray source of between 5 and 50% of the SID.

8. X-ray absorbing mask of any of the previous claims, comprising a large open zone [13] providing between 5-10% of open recess space for imaging a region of special interest.

9. Method to optimize geometric parameters and exposure settings of an X-ray imaging device for a main-shot acquisition that is to be performed on said X-ray imaging device, comprising the steps of:

- positioning the X-ray absorbing mask of Claim 1,
- acquiring a calibration pre-shot image by said X-ray imaging sensor of said X-ray imaging device by exposing said X-ray imaging sensor when exposed by said X-ray source,
- analysing said digital calibration pre-shot image to calculate geometric adjustments,
- analysing said digital calibration pre-shot image to calculate exposure setting adjustments,
- applying said geometric and exposure setting adjustments to the X-ray imaging device,
- removing the X-ray absorbing mask from the X-ray imaging device.

Fig. 1

Fig. 2

Fig. 3

Europäisches
Patentamt

European
Patent Office

Office européen
des brevets

# EUROPEAN SEARCH REPORT

Application Number

EP 20 16 8199

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 104 997 525 B (HEDY MEDICAL DEVICE CO LTD) 13 February 2018 (2018-02-13) | 1-8 | INV. A61B6/00 |
| A | * figure 1 * | 9 | |
| X | US 2012/051523 A1 (FEKE GILBERT [US]) 1 March 2012 (2012-03-01) * figure 1 * | 1,2,5-8 | |
| X | JP 2004 245623 A (FUJI PHOTO FILM CO LTD) 2 September 2004 (2004-09-02) * figures 1,3 * | 1-8 | |
| A,D | US 9 615 803 B2 (GEN ELECTRIC [US]) 11 April 2017 (2017-04-11) * the whole document * | 1-9 | |
| A,D | US 7 488 107 B2 (GEN ELECTRIC [US]) 10 February 2009 (2009-02-10) * the whole document * | 1-9 | |

TECHNICAL FIELDS
SEARCHED      (IPC)

A61B

The present search report has been drawn up for all claims

| Place of search | Date of completion of the search | Examiner |
|---|---|---|
| Munich | 19 August 2020 | Koprinarov, Ivaylo |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 16 8199

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2020

| Patent document cited in search report | | Publication date | Patent family member(s) | | Publication date |
|---|---|---|---|---|---|
| CN 104997525 | B | 13-02-2018 | NONE | | |
| US 2012051523 | A1 | 01-03-2012 | NONE | | |
| JP 2004245623 | A | 02-09-2004 | NONE | | |
| US 9615803 | B2 | 11-04-2017 | CN 106572828 A | | 19-04-2017 |
| | | | EP 3154432 A2 | | 19-04-2017 |
| | | | US 2015359498 A1 | | 17-12-2015 |
| | | | WO 2015195515 A2 | | 23-12-2015 |
| US 7488107 | B2 | 10-02-2009 | CN 1915169 A | | 21-02-2007 |
| | | | US 2007041508 A1 | | 22-02-2007 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9615803 B2
- US 7488107 B2